(19) Europäisches Patentamt
European Patent Office
Office européen des brevets

(11) EP 4 071 181 A1

(12) EUROPEAN PATENT APPLICATION

(43) Date of publication:
**12.10.2022 Bulletin 2022/41**

(21) Application number: **22167378.3**

(22) Date of filing: **08.04.2022**

(51) International Patent Classification (IPC):
***C08B 37/16*** (2006.01) ***C07H 1/00*** (2006.01)
***C07H 3/02*** (2006.01)

(52) Cooperative Patent Classification (CPC):
**C08B 37/0012; C07H 1/00; C07H 3/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.04.2021 US 202163173034 P**

(71) Applicants:
- **Qatar Foundation for Education, Science and Community Development**
  **Doha (QA)**
- **Total E&P Golfe Ltd.**
  **Doha (QA)**

(72) Inventors:
- **FANG, Lei**
  **Doha, Qatar (QA)**
- **PHILLIPS, Bailey**
  **Doha, Qatar (QA)**
- **BANERJEE, Sarbajit**
  **Doha, Qatar (QA)**
- **AL-HASHIMI, Mohammed**
  **Doha, Qatar (QA)**
- **HANDY, Joseph**
  **Doha, Qatar (QA)**
- **KALIN, Alexander**
  **Doha, Qatar (QA)**

(74) Representative: **K&L Gates LLP**
**Markgrafenstrasse 42**
**10117 Berlin (DE)**

# (54) CYCLODEXTRIN-DERIVED POLYMER NANOPARTICLES FOR ADSORPTION AND SYNTHESIS THEREOF

(57) Cross-linked polymer nanoparticles were designed and synthesized with cyclodextrins as the starting monomer. These nanoparticles display exemplary adsorption properties in terms of capacity and selectivity, which comes from the synthetic design. The nanoparticles are also highly robust for various practical adsorption applications. The new synthetic method of these nanoparticle materials involves the reaction of cyclodextrin monomers through an emulsion polymerization approach. The size of the nanoparticles can be tuned easily.



Processed by Luminess, 75001 PARIS (FR)

## Description

BACKGROUND

It is challenging to process commercially available activated carbon into desired well-defined nanoparticles for maximum adsorption performance. Meanwhile, activated carbon exerts no selectivity in the adsorption process, making it unsuitable for sensing or separation applications.

A facile synthetic method is developed to afford cyclodextrin-derived polymer networks that exhibit high selectivity in capturing certain organic compounds in water. The sustainable and scalable synthesis, together with the highly robust adsorption performance enables efficient removal and/or separation of organic molecules from aqueous solution in a continuous flow system.

Selective molecular adsorption is critical to sensing, separation, environmental remediation, and many other applications. During adsorption of small organic molecules, selectivity is often achieved by either specific non-covalent interactions or size-matching between the adsorbent backbone and the adsorbate molecules. Besides selectivity, practical applications often demand the adsorbent materials to be robust in the operational conditions, easy to use and recycle, and inexpensive to produce. Many porous materials, such as covalent organic frameworks, conjugated microporous polymers, and hyper-crosslinked polymers, have been extensively investigated for this purpose. Among these, bottom-up synthesized, crosslinked porous polymer networks are an intriguing class of adsorbents due to their versatile structural tunability, facile synthesis, and resistance to dissolution. Macrocyclic molecules such as calixarenes, pillararenes, cyclodextrins, and crown ethers, have been incorporated into porous polymer networks, with the promise of combining the advantages of supramolecular hosts and porous polymer networks for selective molecular adsorption. These materials have indeed exhibited unprecedented efficiency and selectivity that was not accessible on conventional adsorbent materials, such as activated carbon.

Despite exciting recent advancements achieved in supramolecular host-incorporated adsorbent polymers, it is still challenging to deploy them in large scale applications partially due to the need for inexpensive, efficient, and environmentally friendly synthesis of these materials. From a perspective of macrocyclic starting materials, cyclodextrins are ideal because they can be produced in a sustainable, low cost manner. Cyclodextrins are composed of glucopyranosidic repeating units that possess rich hydroxyl functional groups on the outer side of the macrocycle, enabling potential self-condensation reactions for crosslinking. Once crosslinked into a solid network, the juxtaposition of the hydrophobic cavity and hydrophilic outer side of the macrocycle yields a hydrophilic polymer network that can selectively capture hydrophobic guest molecules inside the cyclodextrin cavity. Herein, by taking full advantage of these features of cyclodextrin, disclosed herein is an extraordinarily facile and scalable synthesis of linker-less cyclodextrin-derived polymer networks (CD-PNs), and their excellent selectivity towards adsorbing organic molecules in aqueous media. This approach affords robust materials, while being highly sustainable and scalable for future industrial application.

SUMMARY

According to one non-limiting aspect of the present disclosure, a method of synthesizing a nanoparticle material involves reaction of cyclodextrin monomers through in-situ emulsion polymerization.

According to another non-limiting aspect of the present disclosure, a nanoparticle material prepared by the above method has built-in binding sites for specific materials.

According to another non-limiting aspect of the present disclosure, an adsorbent comprises the above nanoparticle material.

According to yet another non-limiting aspect of the present disclosure, the above adsorbent can be used for industry chemical separation, environment remediation, environment monitoring, and/or chemical sensing.

One advantage of the disclosed inventions is that, despite the undefined chemo- and regio-selectivity of the reaction, the important macrocyclic constitution of the cyclodextrin unit was expected to be preserved during the crosslinking, so that the resulting CD-PNs inherit the capability of selective guest adsorption from cyclodextrin.

Yet another advantage is that the reaction uses inexpensive and non-toxic MSA as the catalyst and solvent simultaneously, without the addition of any other reagent, ensuring the sustainability and scalability of the synthesis.

Another advantage of the disclosed invention is that the pristine nature of this reaction, involving solvent and monomer, allows for versatile and amenable solution processing of the material through in situ crosslinking into a desired form and morphology, such as well-defined smooth thin films that maintain the structure and performance of the bulk material

Another advantage is that the cyclodextrin monomer is crosslinked directly with no additional spacer affording the CD-PNs with the theoretical potential for a maximized density of macrocyclic binding sites.

Additional features and advantages are described herein, and will be apparent from the following Detailed Description and the figures.

BRIEF DESCRIPT OF THE FIGURES

FIG. 1 shows Scheme. 1: Synthetic scheme and proposed reaction scheme of CD-PN.

FIG. 2 illustrates (a) Schematic representation and digital photograph of column separation using b-CD-PN as the adsorbent (flow rateB0.4 mL s_1); (b) structures of tested small molecular dyes; column dye adsorption data of (c) b-CD-PN (d) a-CD-PN (e) Glu-PN and (f) activated carbon (C = eluent concentration, C0 = feed concentration). In Fig. 2f, all dye adsorption data are overlapping except for Congo red.

FIG. 3 shows Adsorption performance of a mixture of 0.005 mM methylene blue + 0.1 mM rhodamine B by (a) b-CD-PN (b) a-CD-PN (c) Glu-PN and (d) activated carbon. In Fig. 3d, the data points for methylene blue and rhodamine B are overlapping.

FIG. 4 shows (a) Adsorption performance of b-CD-PN to methylene blue after variable chemical treatments (Q = adsorption after treatment, Q0 = baseline adsorption). (b) Recyclability of b-CD-PN methylene blue adsorption where cycle 0 is the first use.

FIG. 5 shows a correlation between the reaction temperature and C1s XPS peak of β-CD and β-CD-PN prepared under varying temperatures.

FIG. 6 shows an XPS C1s C-C deconvoluted peaks of β-CD and β-CD-PN prepared under varying temperatures.

FIG. 7 shows a solid-state CP/MAS 13C NMR spectra of β -CD-PN and β-cyclodextrin.

FIG. 8 shows an FTIR spectra of β -CD-PN compared with β -cyclodextrin starting material.

FIG. 9 shows an FTIR spectra comparison of β -CD-PN and β -CD-PN-Film.

FIG. 10 shows a TGA plot of β -CD-PN compared with β -cyclodextrin starting material in N2 atmosphere.

FIG. 11 shows a CD-PN film fabrication method.

FIG. 12 shows an SEM image of β -CD-PN sample powderized by a commercial coffee grinder.

FIG. 13 shows a top view SEM images of β -CD-PN film.

FIG. 14 shows a cross-section view SEM image of β -CD-PN film.

FIG. 15 shows molecular structures and dimensions of the dyes tested in the study. (a) bisphenol A, (b) methylene blue, (c) rose bengal, (d) rhodamine B, and (e) congo red.

FIG. 16 shows an adsorption efficiency of different organic molecules by β -CD-PN as a function of time.

FIG. 17 shows an adsorption efficiency of different organic molecules by β -CD-PN. The adsorption efficiency is an average of 4 trials.

FIG. 18 shows an isotherm corresponding to the adsorption of methylene blue on β -CD-PN (R2 = 0.9907).

FIG. 19 shows an isotherm corresponding to the adsorption of bisphenol A on β -CD-PN (R2 = 0.9609).

DETAILED DESCRIPTION

All percentages are by weight of the total weight of the composition unless expressed otherwise. Similarly, all ratios are by weight unless expressed otherwise. When reference is made to the pH, values correspond to pH measured at 25 °C with standard equipment. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, unless the context clearly dictates otherwise. Thus, the references "a," "an" and "the" are generally inclusive of the plurals of the respective terms. For example, reference to "an ingredient" or "a method" includes a plurality of such "ingredients" or "methods." The term "and/or" used in the context of "X and/or Y" should be interpreted as "X," or "Y," or "X and Y."

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of and "consisting of the disclosed components. Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

The present technology provides a new synthetic method and the resulting nanoparticle materials. The method involves the reaction of cyclodextrin monomers through a new emulsion polymerization approach. The size of the nanoparticles can be tuned easily by modifying the synthetic method. In-situ emulsion polymerization processing is employed in order to obtain the nanoparticulate shape of the polymer. The disclosed sustainable materials have built in binding sites endowing it with high adsorption selectivity towards specific compounds. The synthetic method is cost effective and requires no reaction partner other than the solvent and surfactant. The method endows the material with a high density of binding sites with the morphological control being key to accessing the binding sites.

A methanesulfonic acid (MSA)-mediated condensation reaction was developed to crosslink b- or a-cyclodextrin monomers, to afford the polymer networks b-CD-PN and a-CD-PN, respectively (Fig. 1). During the synthesis, cyclodextrin monomers were dissolved in MSA and heated at 110 °C for ~48 h without any protection from water or oxygen, followed by simple washing as the work up. In this acid-promoted reaction, the hydroxyl groups on glucopyranoside units from different cyclodextrin monomers undergo ether condensation to crosslink them into a polymer network. The formation of the ether linkages is supported by the emergence of a strong C-O-C stretching peak at 1157 $cm^{-1}$ in the FTIR spectra of CD-PNs (Fig. 8 and Table S2).

In a non-limiting embodiment, at the optimized temperature of 110 °C, the water byproduct is driven out of the solution to push forward the ether condensation via thermodynamic control yielding a fully crosslinked, insoluble product. Other than the dehydrative ether condensation, dehydrative b-elimination also takes place during the reaction, affording sp2-carbons in the product as evidenced by XPS and solid-state NMR (Fig. 6 and 7). Excessive b-elimination is avoided at the optimized temperature of 110 °C in order to prevent the formation of an overly hydrophobic material that would not be favorable for aqueous applications. The extent of dehydration, associated with both ether condensation and b-elimination, was estimated based on the isolated yields and elemental analysis data. Taking b-CD-PN as an example, the isolated yield is calculated to be 64% based on the mass of the monomer, corresponding to ~22.69 mol of water loss per mol of b-cyclodextrin. Comparing the elemental composition of b-CD-PN (found: C, 66.50; H, 4.70; O, 28.00; S, <0.05%) with the starting material, b-cyclodextrin (found: C, 44.45; H, 6.22; O, 49.30%), one can estimate ~19.46 mol of water loss per mol of b-cyclodextrin in the reaction. These values agree well with each other despite the small error in the measurement of yield and elemental analysis. It is worth noting that other strong acid-promoted side-reactions, such as isomerization of the glucopyranosidic building block into a glucofuranoside structure through ring opening and re-annulation, are also possible (Fig. 1).

The simple MSA-promoted reaction carries a number of advantages for practical adsorption applications. First, despite the undefined chemo- and regio-selectivity of the reaction, the important macrocyclic constitution of the cyclodextrin unit was expected to be preserved during the crosslinking, so that the resulting CD-PNs inherit the capability of selective guest adsorption from cyclodextrin. Second, the reaction takes advantage of using inexpensive and non-toxic MSA as the catalyst and solvent simultaneously, without the addition of any other reagent, ensuring the sustainability and scalability of the synthesis. Thirdly, the pristine nature of this reaction, involving solvent and monomer, allows for versatile and amenable solution processing of the material through in situ crosslinking into a desired form and morphology, such as well-defined smooth thin films that maintain the structure and performance of the bulk material (Fig. 9, 11, 13, and 17). Lastly, the cyclodextrin monomer is crosslinked directly with no additional spacer affording the CD-PNs with the theoretical potential for a maximized density of macrocyclic binding sites.

In a non-limiting embodiment, N2 adsorption isotherm of b-CD-PN showed low measurable surface area (<10 $m^2$ $g^{-1}$). Despite this lack of apparent porosity, the presence of macrocyclic binding sites with defined sizes promises CD-PNs to be good selective adsorbent materials for small molecules dissolved in water, as has been noted for other cyclodextrin-based polymers. The adsorption selectivity and capacitance were investigated using model aqueous solutions of small molecular dyes (Fig. 2b). The sizes of the dye molecules were estimated to better understand the adsorption potential of both a- and b-CD-PN (Fig. 15). The CD-PN materials were first ground into powders and subsequently added to the tested dye solution while stirring with UV-vis absorbance spectroscopy being recorded to determine the adsorption efficiency. Organic molecules that are smaller than the b-cyclodextrin cavity, such as bisphenol A (BPA) and methylene blue, are efficiently adsorbed by b-CD-PN with fast adsorption kinetics (Fig. 16), while larger dye molecules, such as rose Bengal, rhodamine B, and Congo red, are not adsorbed. No significant adsorption of the dye molecules was observed for a-CD-PN owing to its smaller cavity size. Although b-CD-PN displayed fast adsorption properties, it is important to note that the kinetics are limited by diffusion, morphology, and particle size, so these measurements may not be completely representative of an intrinsic chemical property of the material. Langmuir adsorption isotherms of b-CD-PN exhibited remarkably high adsorption capacities for methylene blue and BPA (177.8 and 387.9 mg $g^{-1}$, respectively, see Fig. 18 and 19). The BPA adsorption capacity surpasses those reported on most other cyclodextrin-derived polymer networks (Table S3), meaning that the linker-less b-CD-PN has the potential to reach a maximized density of binding sites compared to those requiring a crosslinker during synthesis.

In yet another non-limiting embodiment, the adsorption results, in conjunction with the scalability of the synthesis, means that b-CD-PN can be employed as a filler adsorbent for energy-efficient separation of organic molecules in aqueous solution. Model flow-through separation experiments were conducted in a miniature column using a 1 mL

syringe (Fig. 2a). The model column contained approximately 100 mg of adsorbent. An aqueous feed solution containing a molecular dye or a mixture of dyes was continuously fed through the column with a flow rate of ~0.4 mL $s^{-1}$, while the eluent was collected in fractions for analysis. As expected, b-CDPN exhibits highly efficient adsorption with exemplary selectivity for BPA and methylene blue (Fig. 2c). For BPA, 99% adsorption efficiency was maintained for the full duration of the 60 mL feed solution (0.2 mM). While for methylene blue 99% adsorption efficiency was maintained for the first 32 mL of feed solution with over 90% adsorption efficiency continuing up to 53 mL of feed solution (0.2 mM). The a-CD-PN had minimal adsorption for molecules of all sizes due to its small cavity size (Fig. 2d). BPA and rose Bengal exhibit marginal adsorption. The BPA adsorption can be attributed to hydrogen bonding interactions with BPA by the residual hydroxyl groups on a-CD-PN. The small amount of adsorption of large rose Bengal molecule is likely due to the partial inclusion of the iodide group into the a-cyclodextrin cavity. The result indicates that careful selection of the macrocycle based on cavity size is critical for achieving different levels of selectivity, which can be extended to various adsorbates.

The performance of two control materials, namely, commercial activated carbon and a crosslinked network of glucose (Glu-PN) without the presence of cyclodextrin-like macrocycles, were investigated and compared. Glu-PN was synthesized under the same MSA-mediated conditions using D-(+)-glucose as the starting material. Flow-through adsorption tests on Glu-PN showed almost no uptake of all tested molecules. A small uptake of BPA (Fig. 2e) was observed likely due to hydrogen bonding, agreeing with that observed on a-CD-PN. Commercial activated carbon exhibited vast adsorption uptake of all tested organic molecules without any selectivity (Fig. 2f) as a result of the rich presence of pores with a broad size distribution. These adsorption data demonstrate that the macrocyclic backbone in b-CD-PN is essential for the observed adsorption selectivity and also suggests that the macrocyclic cavity is retained from the cyclodextrin monomers throughout the synthesis despite the strong conditions.

The extent of selective adsorption performance of b-CD-PN was further confirmed by the adsorption of small organic molecules in the presence of a high concentration of interfering non-adsorbing organic molecules. The adsorption of methylene blue (0.005 mM) was tested in the presence of an excess amount of rhodamine B (0.1 mM) (methylene blue : rhodamine B = 1 : 20). b-CD-PN exhibited complete adsorption of methylene blue while adsorbing little to no rhodamine B (Fig. 3a). In comparison, a-CD-PN adsorbed a small amount of methylene blue and very little rhodamine B (Fig. 3b). Glu-PN adsorbed a minimal amount of rhodamine B and no methylene blue (Fig. 3c). Activated carbon, in contrast, displayed no selectivity by completely adsorbing both dyes (Fig. 3d). These data further demonstrate the excellent size selectivity of the b-CD-PN, while also showing its strong affinity for molecules with size and shape complementarity even amongst a substantial amount of interference. While selectivity is retained, the adsorption capacity is somewhat impacted potentially due to intermolecular interactions between the dye molecules. Additionally, it is expected that the adsorption properties can be enhanced with careful morphological control, which can be achieved through in situ crosslinking enabled by the pristine nature of the MSA mediated reaction.

The crosslinked nature of b-CD-PN renders it suitable for column separation applications while mitigating concerns related to sloughing and dissolution, and imparts an enhanced robustness into the polymer network that is not inherent in the cyclodextrin monomers. It showed remarkable chemical resistance with little to no loss in methylene blue adsorption performance after exposure to strong aqueous acid or base, or strong organic solvents (Fig. 4a). b-CD-PN also exhibited exemplary recyclability after regeneration by simply washing with methanol from a saturated adsorbed state. Surprisingly, the adsorption performance was not only maintained but also slightly improved after being regenerated from washing (Fig. 4b), due to a decreased particle size after washing and wearing that rendered the binding sites more accessible to the adsorbate. The simple washing regeneration procedure for b-CD-PN is a low energy process furthering its industrial practicality, especially compared with activated carbons, which typically require very high energy regeneration processes.

The sustainable and scalable synthesis of dehydrated cyclodextrin-derived polymer networks was achieved via a facile MSA-mediated condensation reaction. The retained macrocyclic cavities in b-CD-PN enable its exceptional selective adsorption on organic small molecules that match the cyclodextrin cavity size, such as BPA and methylene blue, even in the presence of a highly concentrated interfering compound. In context, b-CD-PN was compared with other cyclodextrin-derived polymers in terms of BPA adsorption capacities and synthetic conditions (Table S3), demonstrating its high adsorption performance as a result of the linker-less synthetic strategy along with enhanced synthetic sustainability and scalability. Moreover, the crosslinked network imparts a high level of robustness into the materials with both thermal and chemical stability, as well as cyclability through a low energy-consuming regeneration process. The combined advantages of this class of CD-PNs promise their future application in fine chemicals/pharmaceutical separation, sensing, and environmental remediation. Furthermore, it is expected that with careful morphological control, which is enabled by the simplistic synthesis, the CD-PNs present a powerful platform for advancing both fundamental knowledge and applications of cyclodextrin-based materials.

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

EXAMPLES

The following non-limiting examples are experimental examples supporting one or more embodiments provided by the present disclosure.

Example 1. General Method

All starting materials and solvents were obtained from commercial suppliers and used without further purification. Activated charcoal was purchased from Sigma-Aldrich with a 100-mesh particle size for adsorption tests. X-ray photoelectron spectroscopy (XPS) data were obtained using an Omicron XPS/UPS system with Argus detector. Solid-state NMR spectra were obtained on a Bruker Avance 400 MHz spectrometer at magic angle spinning (MAS) rates of 10 kHz with 4 mm CP/MAS probes at room temperature. Fourier transform infrared (FTIR) Spectroscopy was recorded using a Shimadzu IRAffinity-1S spectrometer. UV-visible absorption spectra were recorded on a Shimadzu UV-2600 UV-Vis Spectrophotometer. Field-emission scanning electron microscopic (SEM) images were collected using a JEOL JSM-7500F FE-SEM at 5 kV. Samples were sputter coated in platinum/palladium prior to imaging. Thermogravimetric analysis (TGA) was carried out with a TA Q500 thermogravimetric analyzer at a heating rate of 20°C min-1 from 30°C to 900°C under N2 atmosphere. Elemental analysis was performed by Robertson Microlit Laboratories for elemental CHN analysis.

Example 2. Synthetic Procedures

α -CD-PN and β-CD-PN: Cyclodextrin, either β or α , (15.0 g, 13.2 mmol if β-CD or 5.0 g, 5.1 mmol if α -CD) was dissolved in methanesulfonic acid (150 mL) and pre-reacted for 45 minutes with bath sonication to form a dark red solution. The solution was then heated and maintained at 110°C for 48 h. Afterwards, the solution was cooled in an ice bath and quenched with water. The solid was collected and washed with copious amounts of water. Soxhlet extraction by water was then conducted for 24 h to remove uncross-linked low molecular weight material. Subsequently, the solid was dried in a vacuum oven at 80°C for 24 h. The dried solid, α -CD-PN (3.4 g, 68 % yield) and β-CD-PN (9.6 g, 64 % yield) was then ground into a fine powder with a coffee grinder for further characterization and performance tests. Percent yield based on cyclodextrin monomer mass is defined by *Equation S1.* The number of moles of water lost during the reaction can be estimated from the percent yield based on the assumption that all weight loss is due to water loss. Using this method, it is estimated that 22.69 mol of H2O are lost per mole of β-cyclodextrin monomer and 17.43 mol of H2O are lost per mole of α -cyclodextrin monomer.

Percent yield based on cyclodextrin monomer mass is defined by *Equation S1.* The number of moles of water lost during the reaction can be estimated from the percent yield based on the assumption that all weight loss is due to water loss. Using this method, it is estimated that 22.69 mol of H2O are lost per mole of β-cyclodextrin monomer and 17.43 mol of H2O are lost per mole of α-cyclodextrin monomer.

$$\%\ \text{yield} = \left(\frac{\textit{mass of isolated CD} - \textit{PN}}{\textit{mass of CD monomer}} x\ 100\right) \qquad (\textit{Equation S1})$$

Scheme S2. Synthesis of Glu-PN via methanesulfonic acid-mediated condensation.

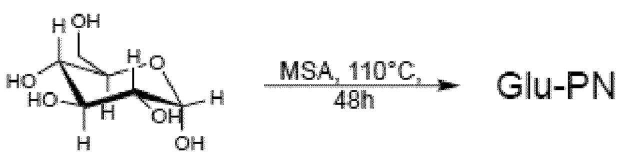


Glucose-Derived Polymer Network (Glu-PN): d-(+)-Glucose (5.0 g, 27.7 mmol) was dissolved in methanesulfonic acid (50 mL) and pre-reacted for 45 minutes under bath sonication. The solution was then heated and maintained at 110°C for 48 h. Afterwards, the solution was cooled in an ice bath and quenched with water. The solid was collected and washed with copious amounts of water. Soxhlet extraction by water was then conducted for 24 h to remove uncross-linked low molecular weight material. Subsequently, the solid was dried in a vacuum oven at 80°C for 24 h. The dried solid, Glu-PN (4.3 g, 86 % yield) was then ground into a fine powder with a coffee grinder for further characterization and performance tests.

Example 3. X-Ray Photoelectron Spectroscopy

In order to monitor how the reaction temperature impacts the level of dehydration in the product, three batches of β-CD-PN were synthesized with the reaction temperature varied at 50°C, 80°C, and 110°C. XPS was used to analyze the nature of carbon in the material. The shift of the C1s C- C peak towards 284 eV corresponding to *sp2* carbon indicated that dehydration via acid-mediated elimination occurred along with cross-linking during the reaction. The content of *sp2* carbon increased as the temperature was elevated as evidenced by a consistent shift of the C1s C-C peak towards 284 eV (Fig. 6).

Table S1. Table of XPS C1s peak positions and areas for β-CD and β-CD-PN prepared under varying temperatures.

| | β-CD | | β-CD-PN 50°C | | β-CD-PN 80°C | | β-CD-PN 110°C | |
|---|---|---|---|---|---|---|---|---|
| Peak | Position | Peak Area | Position | Peak Area | Position | Peak Area | Position | Peak Area |
| C-C | 284.7 | 17.92 | 284.6 | 48.00 | 284.4 | 58.30 | 284.1 | 49.30 |
| C-O | 286.3 | 68.89 | 285.9 | 44.18 | 285.7 | 38.10 | 285.5 | 39.50 |
| C=O | 287.9 | 12.19 | 288.9 | 7.83 | 288.8 | 3.60 | 288.1 | 11.20 |

Example 4. Solid-State NMR

Solid-state 13C CP/MAS NMR of β-CD-PN and β-cyclodextrin were measured at room temperature in the powder form. In the β-CD-PN spectrum, the emergence of peaks ranging from ~180 - 215 ppm indicate the presence of carbonyls within the polymer, which is not observed in β-cyclodextrin (Fig. 7). Additionally, a peak around 155 ppm indicates the presence of alkene *sp2* carbons in β-CD-PN as observed in XPS (Fig. 6).

Example 5. Fourier Transform Infrared Spectroscopy

FTIR spectra and table of peak assignments is shown below for β-CD-PN and β-cyclodextrin. The emergence of peaks indicative of *sp2* carbons are consistent with the dehydration observed from NMR and XPS (Fig. 6 and 7). Additionally, the FTIR spectra of β-CD-PN and β-CD-PN-Film are compared in Fig. 8 demonstrating that the structure and functional groups of the bulk powder material and the thin film are the same.

Table S2. FTIR peak assignments of β-CD-PN and β-cyclodextrin.

| | β-CD-PN | β-Cyclodextrin |
|---|---|---|
| Functional Group | Absorption ($cm^{-1}$) | Absorption ($cm^{-1}$) |
| C-O-H | 1039 | 1024 |
| C-O-C | 1157 | 1151 |
| C=C | 1658 | - |
| C=O | 1710 | - |
| C=C-H | 3101 | - |
| O-H | 3400 | 3336 |

Example 6. Thermogravimetric Analysis

Thermogravimetric analysis (TGA) of β -CD-PN and β -cyclodextrin starting material are shown below. TGA was conducted in a N2 atmosphere. The dehydration of β -CD-PN leads to an enhanced carbonization yield of β -CD-PN (47%) compared with the β -cyclodextrin starting material (14%).

Example 7. Elemental Analysis Calculation

The extent of dehydration was estimated based on elemental analysis data from β -CD-PN and β - cyclodextrin. The empirical formula of β -CD-PN was determined based on the elemental analysis results, while the degree of dehydration was estimated based on the assumption of no carbon loss during the reaction. Using the respective empirical

formulas, the amount of hydrogen and oxygen lost was determined and listed in *Equation S2.*

$$C_6H_{10}O_5 \rightarrow C_6H_{5.07}O_{1.90} + H_{4.93} + O_{3.10} \qquad \text{(Equation S2)}$$

The extent of hydrogen and oxygen lost was averaged to give an estimation of ~2.78 moles of water lost per glucopyranosidic unit, *i.e.,* ~19.46 moles of water lost per mole of β - cyclodextrin.

Example 8. Film Fabrication

β -/a-CD-PN-film: Cyclodextrin, either β - or α-, (0.2 g, 1.8 mmol if β -CD or 2.1 mmol if α-CD) was dissolved in methanesulfonic acid (2 mL) and pre-reacted for 30 min with bath sonication, forming a dark red solution. The solution was then heated for 3 h at 110 °C. The reaction time was tailored according to the volume of the reaction solution, in order to obtain a viscous yet fluid solution for casting. After the appropriate reaction time, the solution is cooled and cast onto a glass substrate using a glass pipet. Bubbles should be removed if possible. Two pieces of micro cover glass divider with a thickness between 0.12-0.17mm are placed on either side of the substrate (Fig. 9). Then another piece of glass is slowly placed on top. The sandwiched system was further heated at 110 °C for 48 h. Afterwards, the glass on top was carefully removed leaving the film on the bottom glass substrate. The film was subsequently washed with copious amounts of water.

Example 9. Scanning Electron Microscopy

SEM samples were coated with 3 nm of platinum/palladium, 80% and 20%, respectively, using a Cressington Sputter Coater 208 HR for high resolution FE-SEM coating to make the samples conductive. SEM images of the β -CD-PN sample powderized by a commercial coffee grinder and the thin films as described in Section 7 were recorded.

Example 10. Structure Size Modeling

The molecular structures of the dyes were modeled using ChemDraw3D. The molecule sizes were measured on ChemDraw3D between particular atoms within the molecule to give a general idea of the molecular dimensions. These measurements were validated by comparing some of these data with single crystal structures from Cambridge Crystallographic Data Centre analyzed with Mercury crystallography analysis software, which showed percent difference between 0.4~8.5%.

Examples 11.1-11.7 Adsorption Performance

Example 11.1 Adsorption Rate

Powder of β -CD-PN (12 mg) was added into an aqueous solution of organic compounds (12 mL, 0.1 mM). As the solution was stirred, 2 mL aliquots were removed at the time intervals of 10 seconds, 30 seconds, 1 minute, 2 minutes, 5 minutes, and 10 minutes, respectively. The aliquots were passed through a syringe filter to remove the solid CD-PN. The adsorption efficiency was determined by comparing the UV-vis absorbance of these sample with that of the initial solution *(Equation S3),* where C = sample concentration and C0 = initial concentration. The organic compounds tested were methylene blue, bisphenol A, rose Bengal, rhodamine B, and Congo red. The β -CD-PN demonstrated fast and selective adsorption of smaller organic molecules, *i.e.,* methylene blue and bisphenol A, within 10 minutes. Adsorption efficiencies of all the other larger organic molecules were lower than 10% after 10 minutes.

$$Adsoprtion\ Efficiency\ (\%) = \left(1 - \frac{C}{C_0}\right) X100 \qquad \text{(Equation S3)}$$

Example 11.2 β -CD-PN Film Solution Adsorption

β -CD-PN film pieces (15 mg) were added into an aqueous solution of organic compounds (15 mL, 0.1 mM). The solution was stirred for 30 minutes, and then a sample was passed through a syringe filter to remove any small pieces of film that broke off during the stirring. The removal efficiency of the β -CD-PN film was determined as described in Section 11.1. The organic compounds tested were methylene blue, bisphenol A, rose Bengal, rhodamine B, and Congo red. The selectivity is maintained in the film with bisphenol A and methylene blue being adsorbed, while larger

molecules are adsorbed to a much lesser amount. Although the selectivity is still observed, the larger dye molecules were adsorbed more so to the film than was observed for the bulk powder (Fig. 17). This is a result of physical adsorption to the film surface rather than into the pores derived from β -cyclodextrin.

Example 11.3 Adsorption Isotherm

The adsorption isotherms were obtained by adding β -CD-PN (15 mg) to either a methylene blue or bisphenol A solution in water (20 mL) and stirred for 24 h. UV-visible absorption spectra of the solution were recorded to monitor concentration before and after adsorption. The initial concentrations of the solutions were varied from 0.05 to 2.0 mM. The adsorption isotherms were fitted using the Langmuir-Freundlich adsorption isotherm *(Equation S4),* where *Qe* (mg/g) is the amount of dye adsorbed at equilibrium, *Ce* (mg/g) is the equilibrium solute concentration, *Qmax* (mg/g) is the maximum adsorption capacity, *b* is the Langmuir equilibrium constant, and *n* is the Freundlich heterogeneity index. The Langmuir-Freundlich isotherm was chosen to characterize the isotherms because it is applicable to both homogeneous and heterogeneous materials, while also describing the adsorption capacity. The CD-PN materials are expected to have a certain level of heterogeneity due to the myriad of reactions that can occur during synthesis (e.g., ring-opening, β -elimination) leading the Langmuir-Freundlich isotherm to be a better fit than the Langmuir isotherm.

$$Q_e = \frac{Q_{max} b {C_e}^{1-n}}{1 + b {C_e}^{1-n}} \qquad (Equation\ S4)$$

Example 11.4 Column Adsorption

An adsorption column was prepared in a syringe (1 mL) by placing a small piece of cotton at the bottom, followed by adding ~100 mg of α-/β-CD-PN, Glu-PN, or activated carbon as the stationary phase. An aqueous dye solution (0.2 mM) was then prepared. The solution was passed through the column driven by pressurized air with a flow rate of approximately ~0.4 mL/s and an empty bed contact time of approximately ~0.5 seconds. The eluent was collected as fractionated samples (3.5 mL each). The dye concentration in each eluent sample was determined using UV-visible absorption spectroscopy. The amount of dye adsorbed was then calculated based on the concentration difference between the stock solution and eluent. The samples for methylene blue, Congo red, and rose Bengal were diluted before the absorbance measurement in order to fulfill the Beer-Lambert law.

Example 11.5 Column Adsorption with Interference

A column was prepared as described above using β -CD-PN. A mixed rhodamine B and methylene blue solution in water was prepared (0.1 mM rhodamine B + 0.005 mM methylene blue). The mixed solution was passed through the columns as described in Section 11.4. The eluent was collected and measured with UV-visible absorption spectroscopy as described above.

Example 11.6 Recyclability

β -CD-PN (20 mg), either virgin or recycled, was stirred in a solution of methylene blue (20 mL, 0.1 mM) for 1 h. The amount of adsorbed methylene blue was determined by comparing the light absorbance of the solution before and after β -CD-PN treatment. After each test, the β -CD-PN was stirred in 30 mL of methanol at 35°C for 2 h to cleanse the adsorbed methylene blue, and subsequently collected via centrifugation, followed by washing and drying. The recycled sample was then dried under vacuum before use in the next cycle.

Example 11.7 Solvent Resistance

β -CD-PN (100 mg) was suspended and stirred in each of the following solvent/solution for 1 week: 0.1 M HCl, 0.1 M NaOH, Methanol, dichloromethane, hexanes, or acetone. Afterwards, the suspended solid was collected by centrifugation, washed, and dried under vacuum. Materials soaked in HCl or NaOH solutions were washed thoroughly with water before drying. After drying, the treated β -CD-PN (12 mg) was added into a methylene blue solution (12 mL, 0.1 mM) and stirred for 24 h. The methylene blue adsorption amount was then measured by UV-visible absorbance before and after the treatment.

Example 12. Cyclodextrin-Based Polymer Comparison

The structure, synthetic conditions, and BPA adsorption capacity are compared for a series of cyclodextrin-based polymers. The prime difference between the β - CD-PN polyme and other cyclodextrin based polymers is that it is linker-less, meaning the cyclodextrin is directly crosslinked with no additional crosslinker. Additionally, the synthetic conditions are considerably greener than many of the other cyclodextrin-based polymers. The BPA adsorption capacity of β -CD-PN is significantly higher than other cyclodextrin-based polymers. The comparison supports the theory that the linker-less CD-PN polymer has the potential for an enhanced binding site density, which enables it with a high adsorption capacity from cyclodextrin itself.

Table S3. Comparison of crosslinked cyclodextrin-based polymers.

| Polymer | Monomer | Crosslinker | Conditions | CD Functional-ization | BPA $Q_{max}$ (mg/g) | Citation |
|---|---|---|---|---|---|---|
| CD-PN | β-CD | N/A | MSA, 110°C, 48 h | Dehydrated | 386 | **This Work** |
| P-CDP | β-CD | 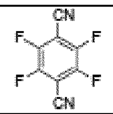 | $K_2CO_3$, THF, 80 °C, 48 h | N/A | 88 | Alsbaiee[2] |
| TFN-CDP-2 | β-CD | 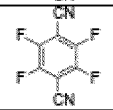 | $K_2CO_3$, DMSO, 80 °C, 18 h | Phenolated | 250 | Klemes[3] |
| P-CDEC | β-CD | 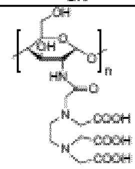 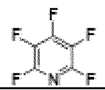 | 8:1 MeTHF: H2O, $K_2CO_3$, 80 °C, 48 h | N/A | 59-66* | Yu[4] |
| β-CD COF | heptakis(6-amino-6-deoxy)-β-CD | 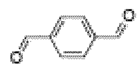 | 50:50 $EtOH:H_2O$, AcOH, r.t., 48 h | Aminated | 20 | Wang[5] |
| β-CD NCP | heptakis(6-amino-6-deoxy)-β-CD | 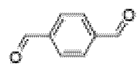 | 50:50 $EtOH:H_2O$, Ammonia, r.t., 48 h | Aminated | 10 | Wang[5] |
| P-CD-P5A-P | β-CD, Pillar[5]arene | 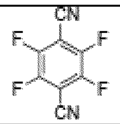 | $K_2CO_3$, THF, 80 °C, 72 h | N/A | 258 | Lu[6] |
| T-E-CDP | β-CD | 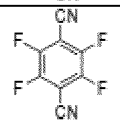 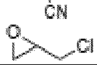 | $NaOH_{(aq)}$, 90°C, 3 h | N/A | 128* | Xu[7] |
| ECP | β-CD | 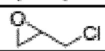 | NaOH | N/A | 84 | Morin-Crini[8] |
| PEGCDP | β-CD | 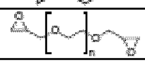 | $NaOH_{(aq)}$, 60°C, 5 h | N/A | 71 | Kono[9] |
| EGCDP | β-CD | 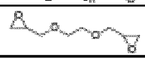 | $NaOH_{(aq)}$, 60°C, 5 h | N/A | 78 | Kono[9] |
| β-CDP | β-CD | 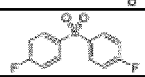 | Toluene, DMAc, $K_2CO_3$, 150°C, 12 h | N/A | 113 | Wang[10] |
| CD-CA-g-PDMAEMA | β-CD, 2-dimethylamino ethyl methacrylate (DMAEMA) | 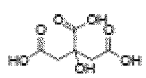 | 1) $KH_2PO_4$, 140°C, 3 h 2) $K_2S_2O_8$, 80°C, 30 min 3) 1 M HCl 4) DMAEMA, 80°C, 3 h | N/A | 79 | Zhou[11] |
| MP-CDP (DFP-CDP)[12] | β-CD | 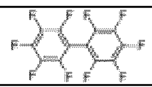 | $K_2CO_3$, 1:3 THF:DMF, 85 °C, 72 h | N/A | 79 | Li[13] |
| CDW7-Triazine | β-CD | 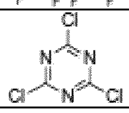 | NaOH, BDMHAC, $CH_3CN$, $H_2O$ | N/A | 57 | Wang[14] |

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

## Claims

1. A method comprising:

   dissolving at least one of β- or α- cyclodextrin in methanesulfonic acid to form a solution,
   heating and maintaining the solution at a temperature above about 100°C,
   quenching the solution to collect a solid, and
   drying the solid in a vacuum oven at a temperature from about 50°C to about 100°C.

2. The method of claim 1, wherein the concentration of β-cyclodextrin is about 13.2 mmol; or wherein the concentration of α-cyclodextrin is about 5.1 mmol.

3. The method of claim 1, wherein the at least one of β- or α- cyclodextrin and the methanesulfonic acid are pre-reacted with bath sonication to form a dark red solution.

4. The method of claim 1, wherein uncross-linked low molecular weight material is removed.

5. The method of claim 1, wherein a yield of the dried solid is about 68% when using α-cyclodextrin and about 64 % when using β-cyclodextrin.

6. The method of claim 1, further comprising grinding the solid into a powder.

7. A method comprising:

   dissolving d-(+)-Glucose in methanesulfonic acid to form a solution,
   heating and maintaining the solution at a temperature above about 100 °C,
   quenching the solution to collect a solid, and
   drying the solid in a vacuum oven at a temperature from 50°C-100°C.

8. The method of claim 7, wherein the concentration of d-(+)-Glucose is about 27.7 mmol.

9. The method of claim 7, wherein the d-(+)-Glucose and methanesulfonic acid are pre-reacted under bath sonication.

10. The method of claim 7, wherein uncross-linked low molecular weight material is removed.

11. The method of claim 7, wherein a yield of the dried solid is about 86%.

12. The method of claim 7, further comprising grinding the solid into a powder.

13. A method comprising:

    dissolving at least one of β- or α- cyclodextrin in methanesulfonic acid to form a solution,
    heating and maintaining the solution at a temperature above about 100°C,
    casting the solution onto a substrate,
    placing at a first micro cover glass divider on a first side of the substrate and a second micro cover glass divider on a second side of the substrate to form a system,
    heating the system at a temperature above about 100°C,
    removing at least one of the first or second micro cover glass divider, and
    washing the film.

14. The method of claim 13, wherein the concentration of β-cyclodextrin is about 1.8 mmol; or wherein the concentration of α-cyclodextrin is about 2.1 mmol; or wherein the at least one of β- or α- cyclodextrin and the methanesulfonic acid are pre-reacted with sonication to form a dark red solution.

15. The method of claim 13, wherein the first and second micro cover glass dividers each have a thickness between about 0.12mm and about 0.17mm.

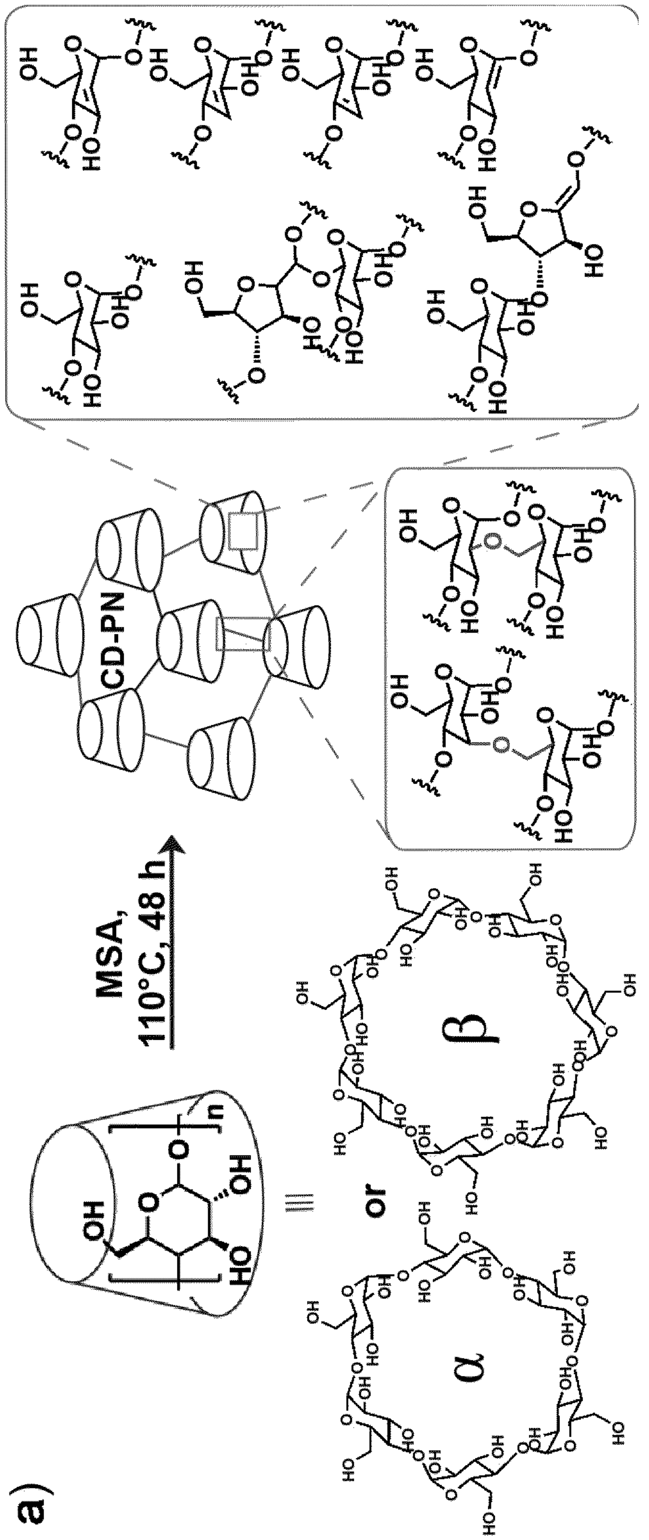


Fig 1

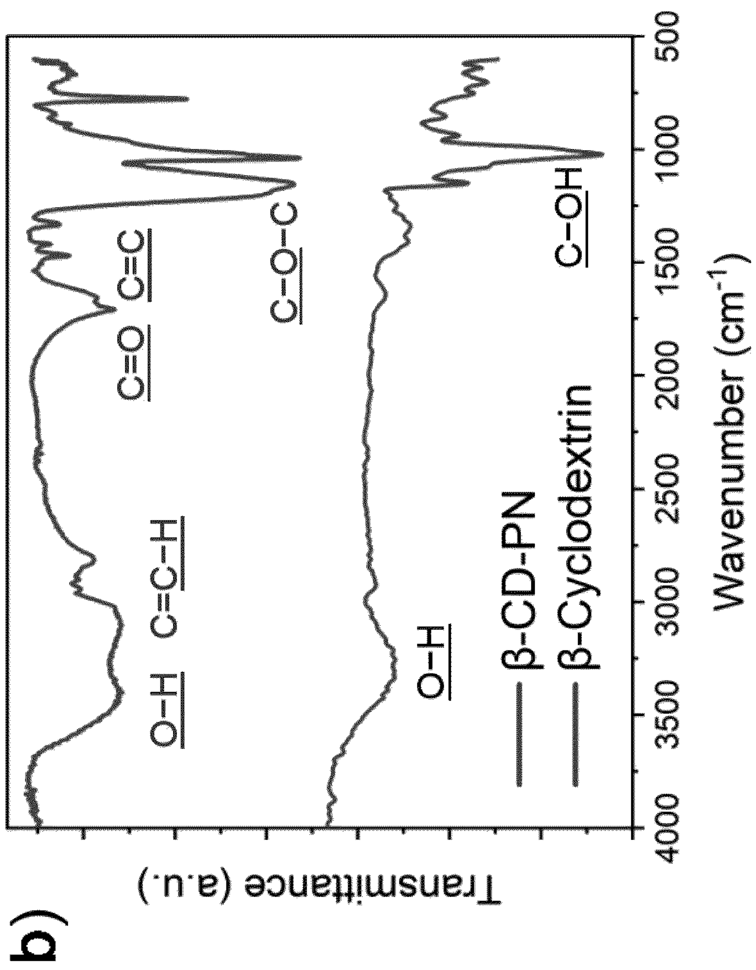


Fig 1
continued

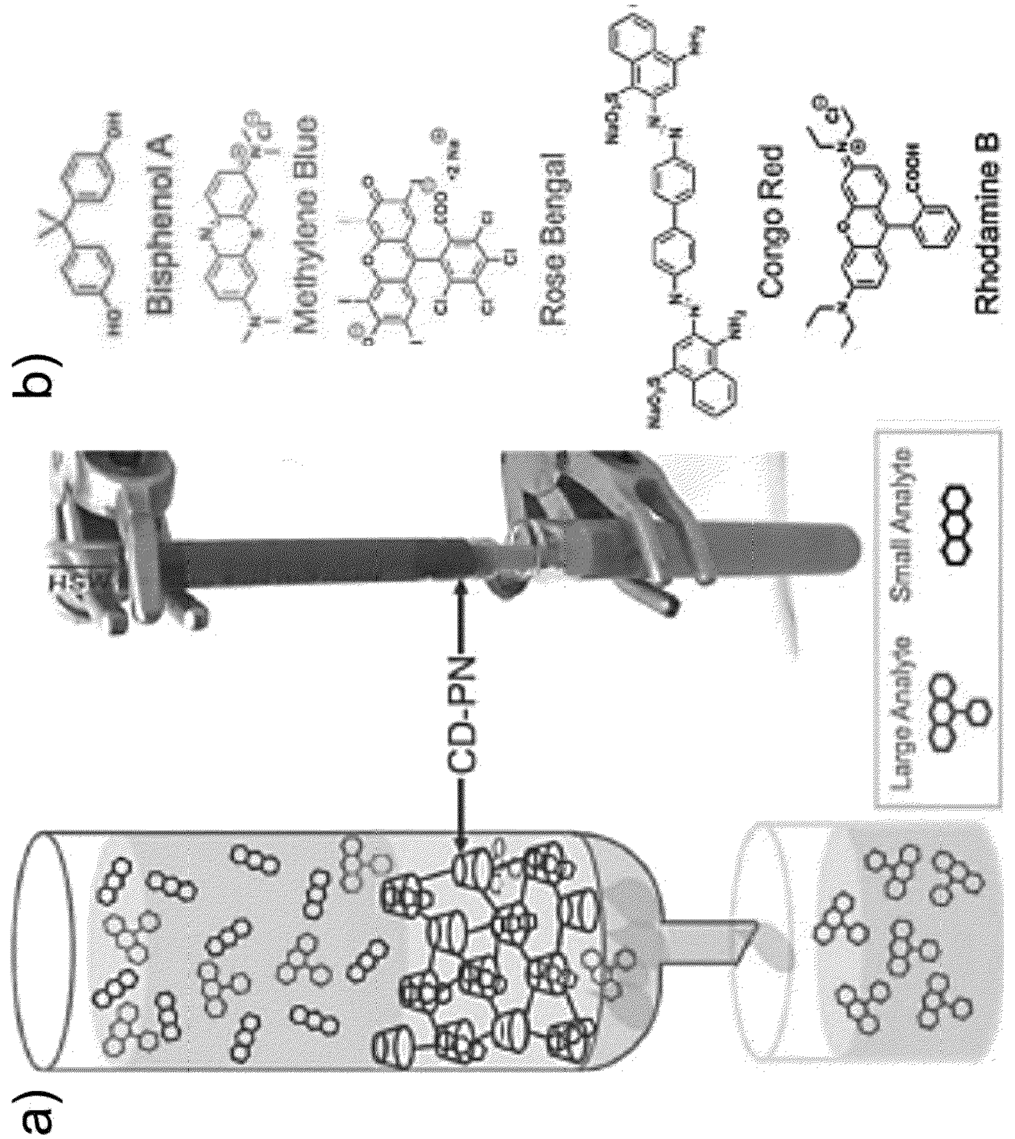
a)
CD-PN
Large Analyte
Small Analyte
b)
Bisphenol A
Methylene Blue
Rose Bengal
Congo Red
Rhodamine B

Fig 2

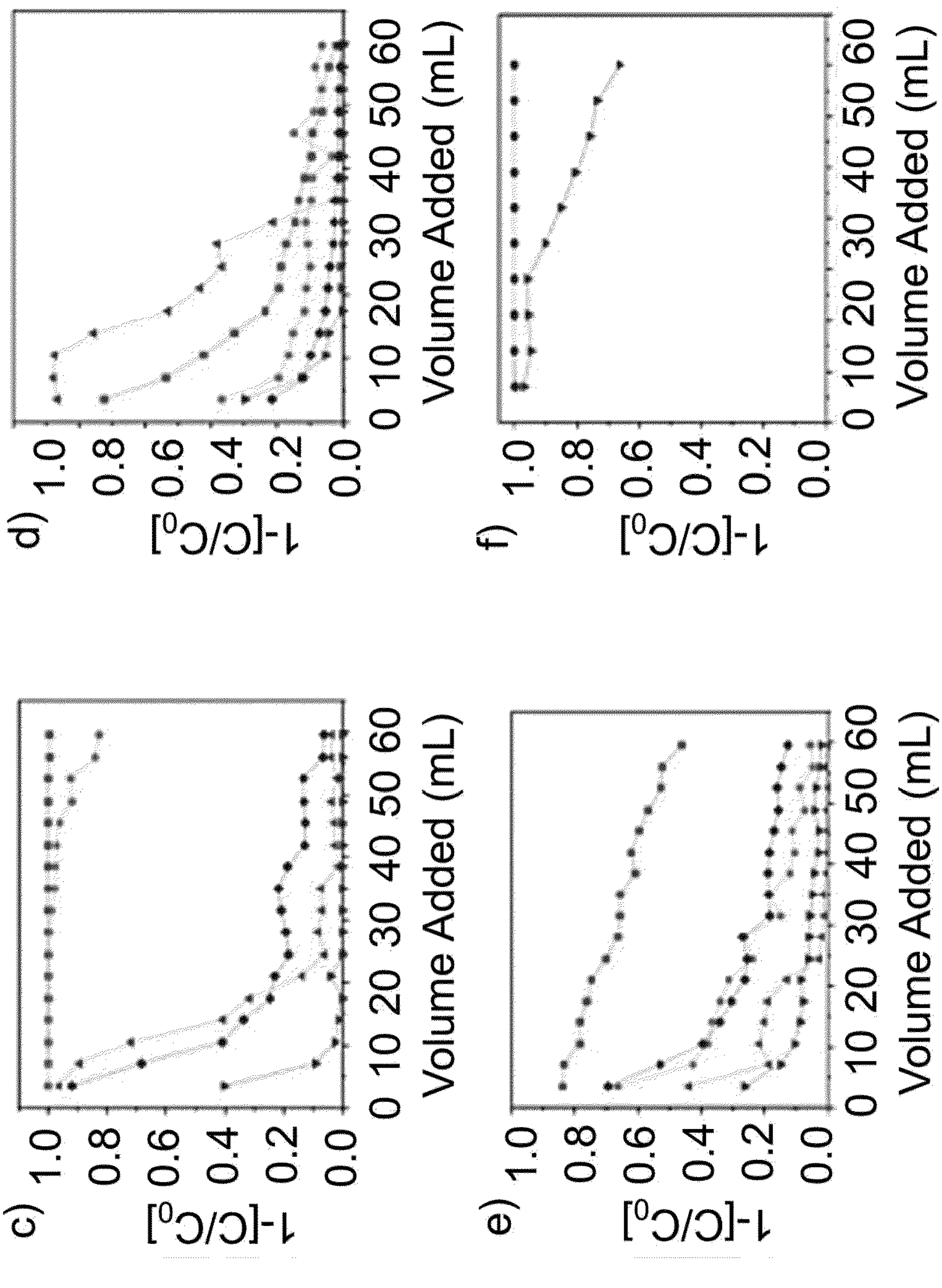


Fig 2
continued

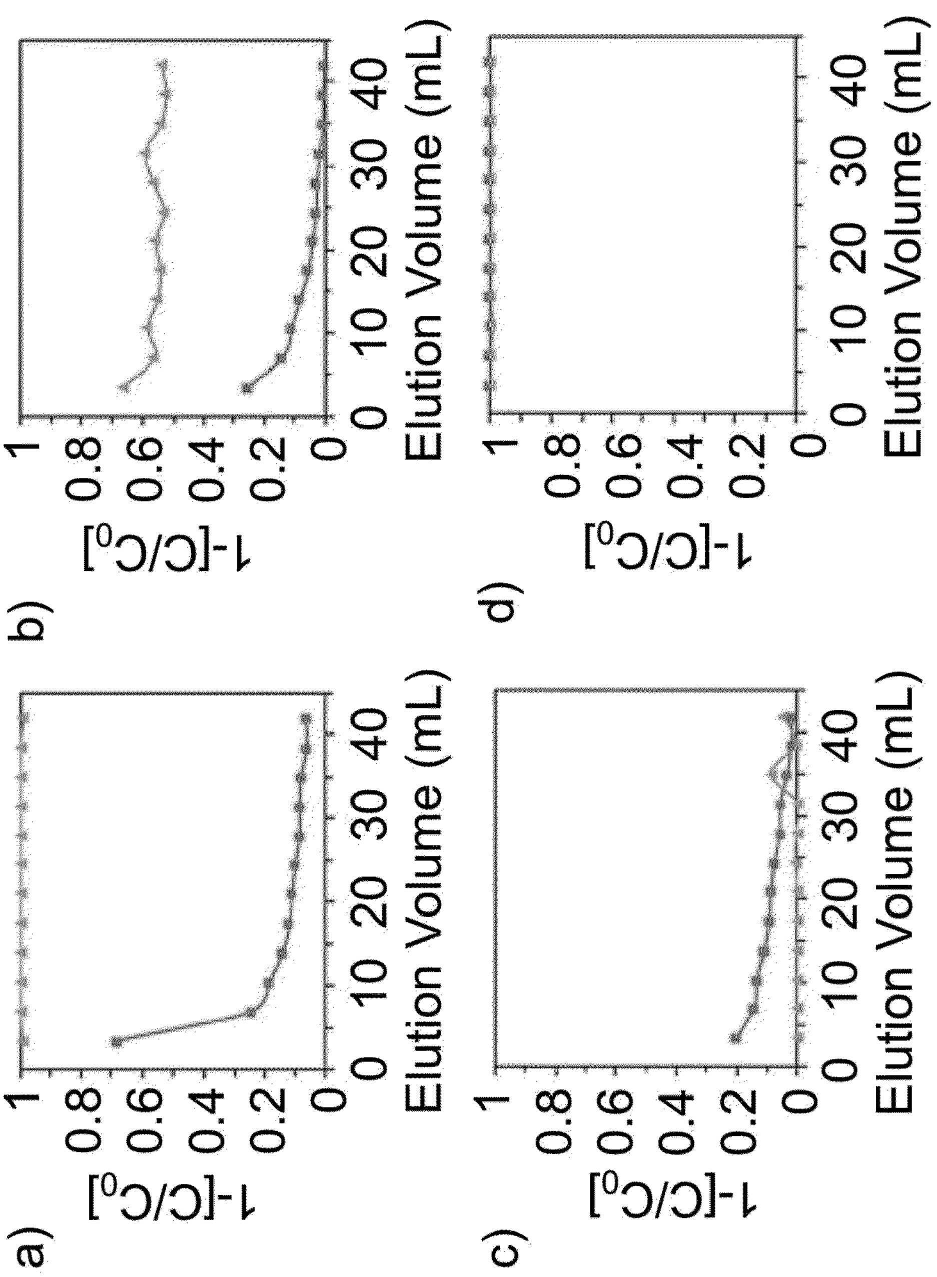
a)
1-[C/C0]
1
0.8
0.6
0.4
0.2
0
0 10 20 30 40
Elution Volume (mL)
b)
1-[C/C0]
1
0.8
0.6
0.4
0.2
0
0 10 20 30 40
Elution Volume (mL)
c)
1-[C/C0]
1
0.8
0.6
0.4
0.2
0
0 10 20 30 40
Elution Volume (mL)
d)
1-[C/C0]
1
0.8
0.6
0.4
0.2
0
0 10 20 30 40
Elution Volume (mL)

Figure 3

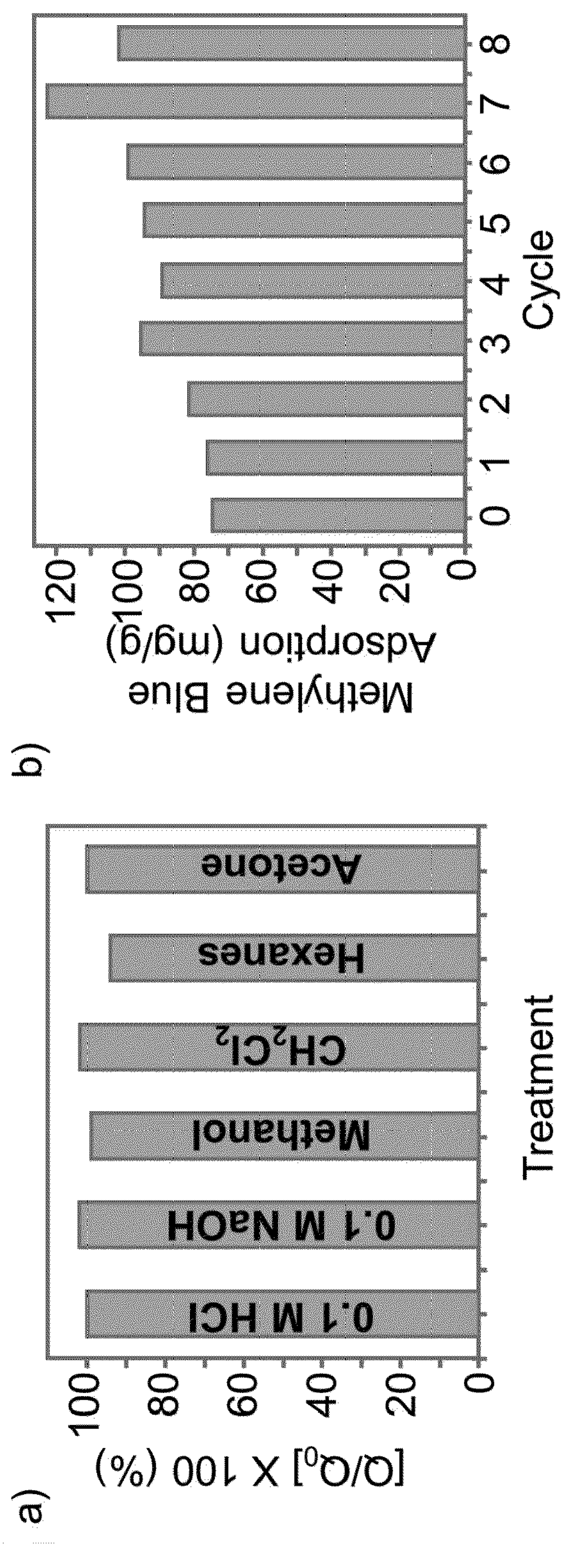
a)
[Q/Q0] X 100 (%)
0.1 M HCl
0.1 M NaOH
Methanol
CH2Cl2
Hexanes
Acetone
Treatment
b)
Methylene Blue
Adsorption (mg/g)
Cycle

Figure 4

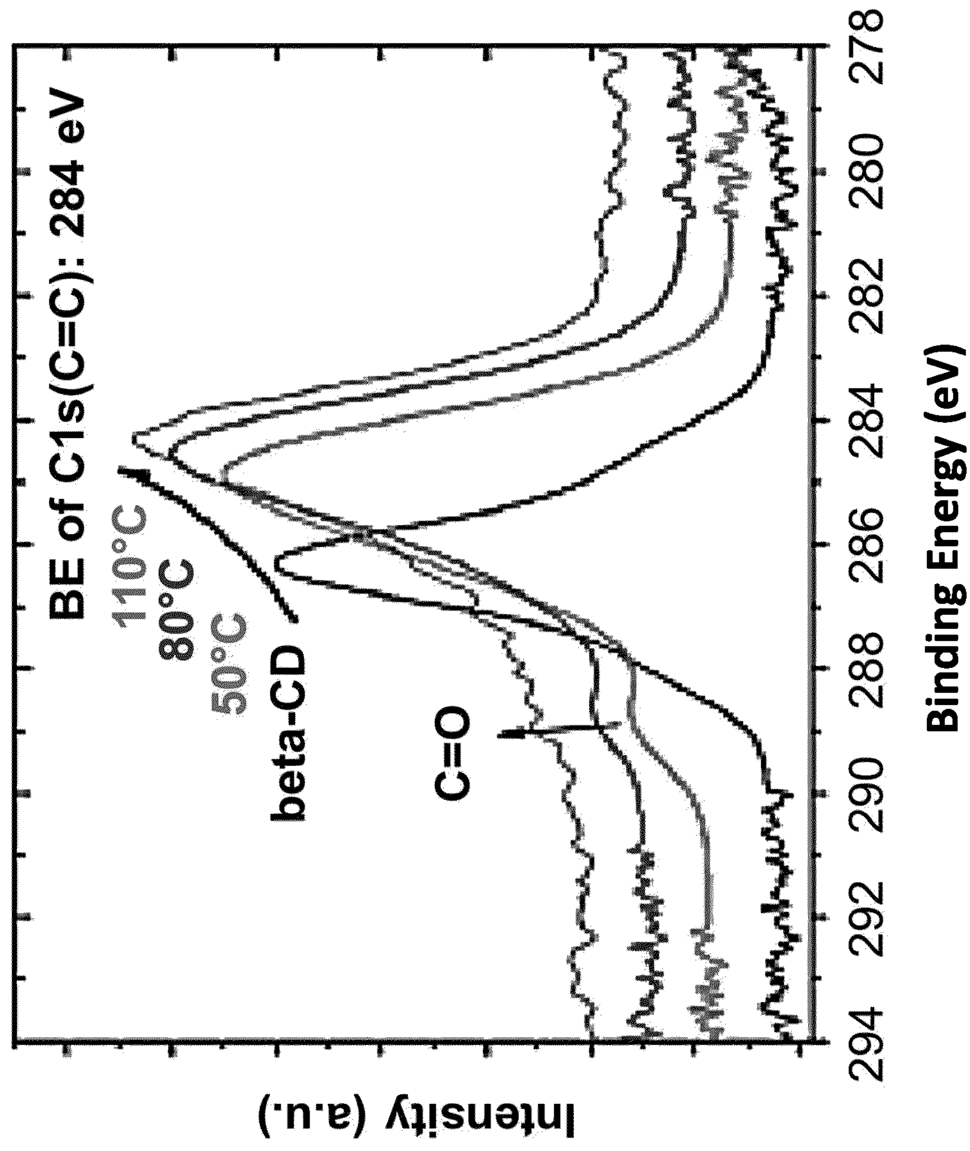
BE of C1s(C=C): 284 eV
110°C
80°C
50°C
beta-CD
C=O
Intensity (a.u.)
Binding Energy (eV)
294
292
290
288
286
284
282
280
278

Figure 5

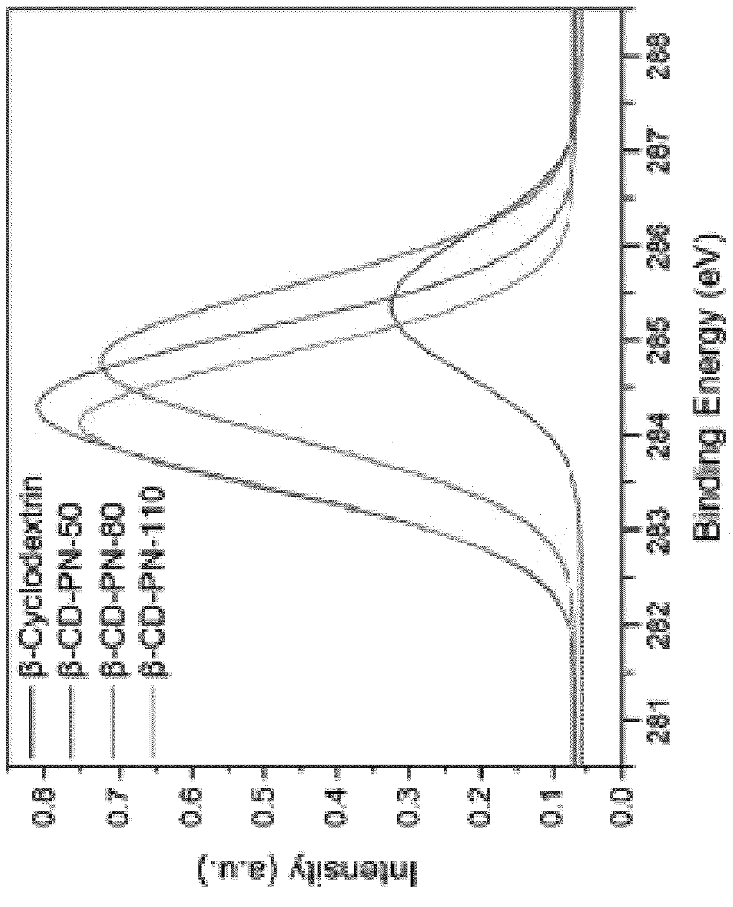

β-Cyclodextrin
β-CD-PN-50
β-CD-PN-80
β-CD-PN-110
Intensity (a.u.)
0.0
0.1
0.2
0.3
0.4
0.5
0.6
0.7
0.8
281
282
283
284
285
286
287
288
Binding Energy (eV)


Figure 6

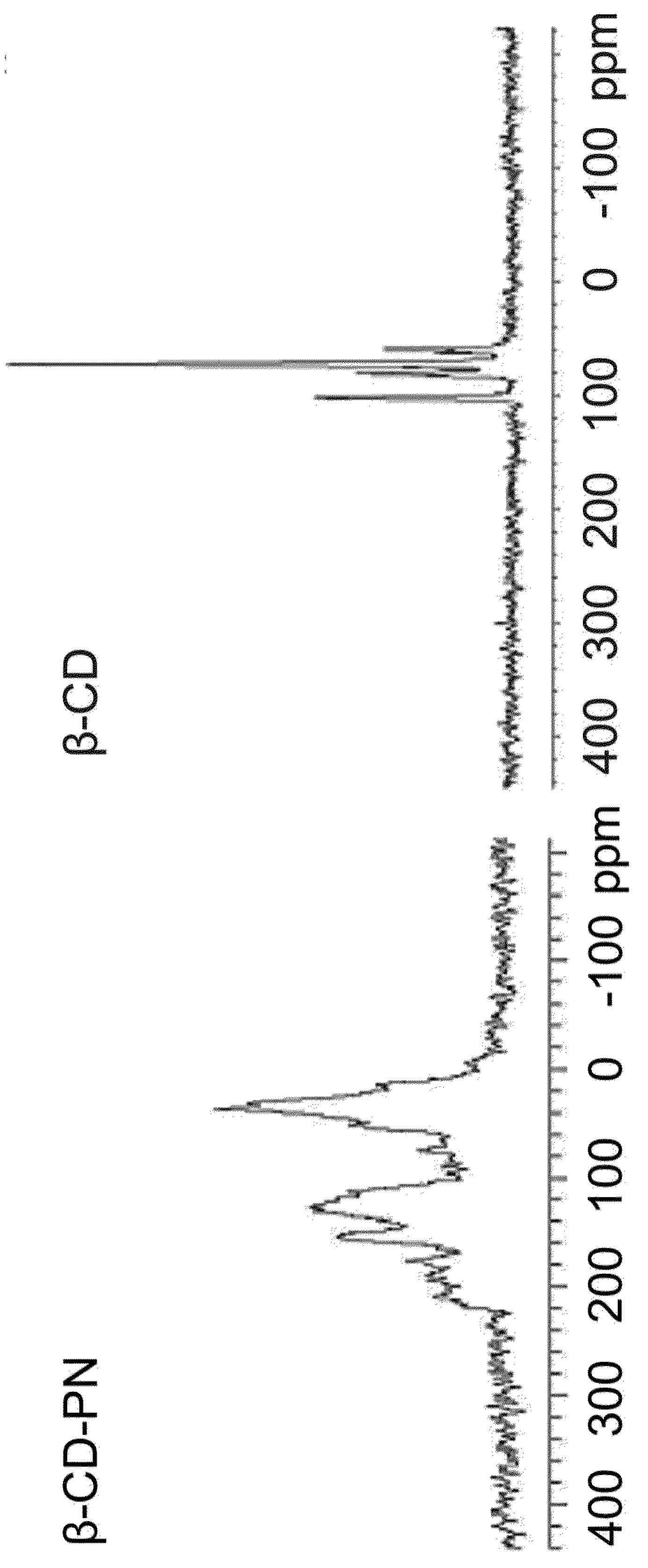


Figure 7

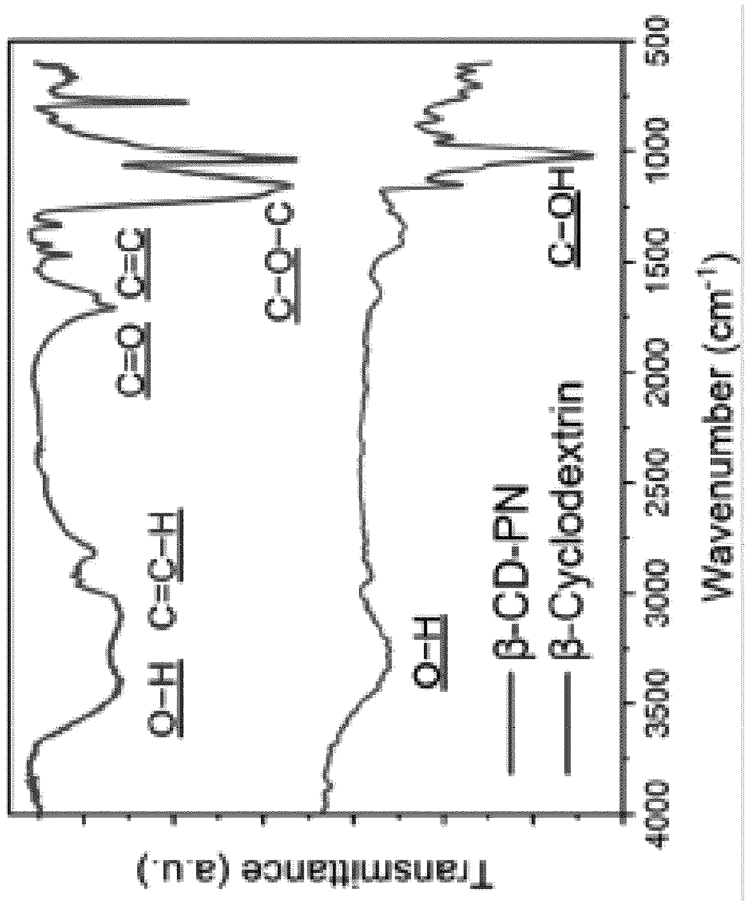
Transmittance (a.u.)
Wavenumber (cm$^{-1}$)
4000
3500
3000
2500
2000
1500
1000
500
O–H
C=C–H
C=O
C=C
C–O–C
O–H
C–OH
β-CD-PN
β-Cyclodextrin

Figure 8

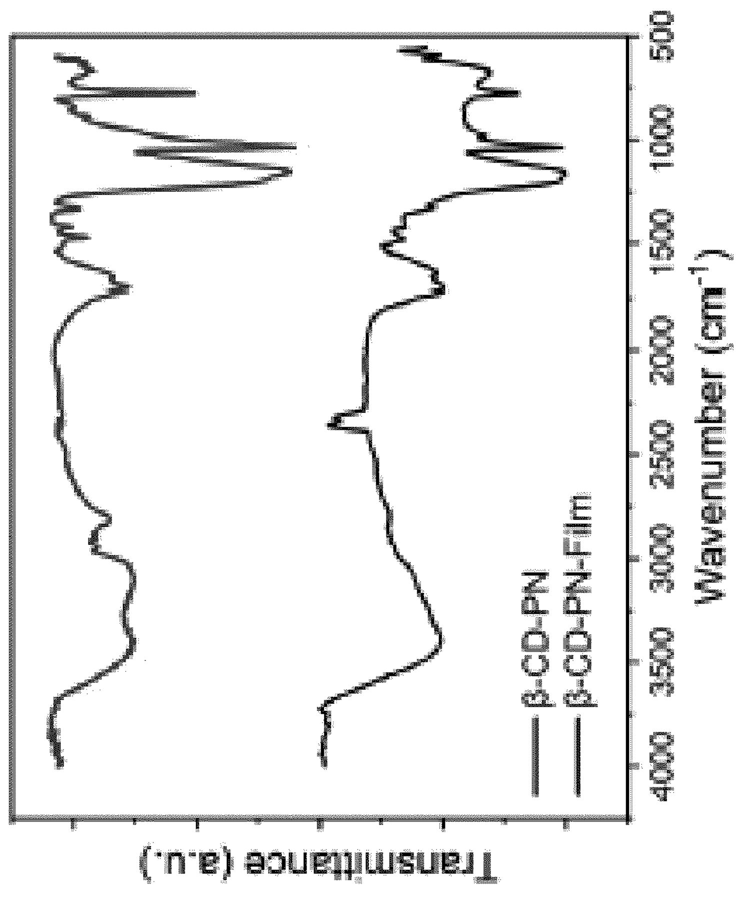
β-CD-PN
β-CD-PN-Film
Transmittance (a.u.)
Wavenumber (cm-1)
4000
3500
3000
2500
2000
1500
1000
500

Figure 9

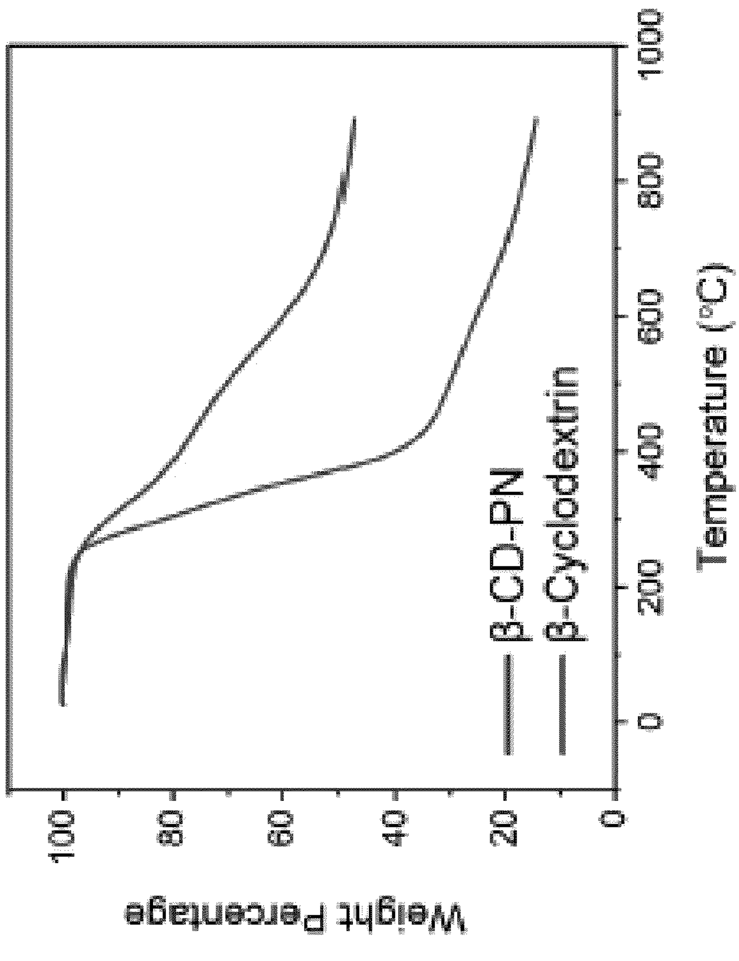

Weight Percentage
Temperature (°C)
β-CD-PN
β-Cyclodextrin
0
20
40
60
80
100
0
200
400
600
800
1000


Figure 10

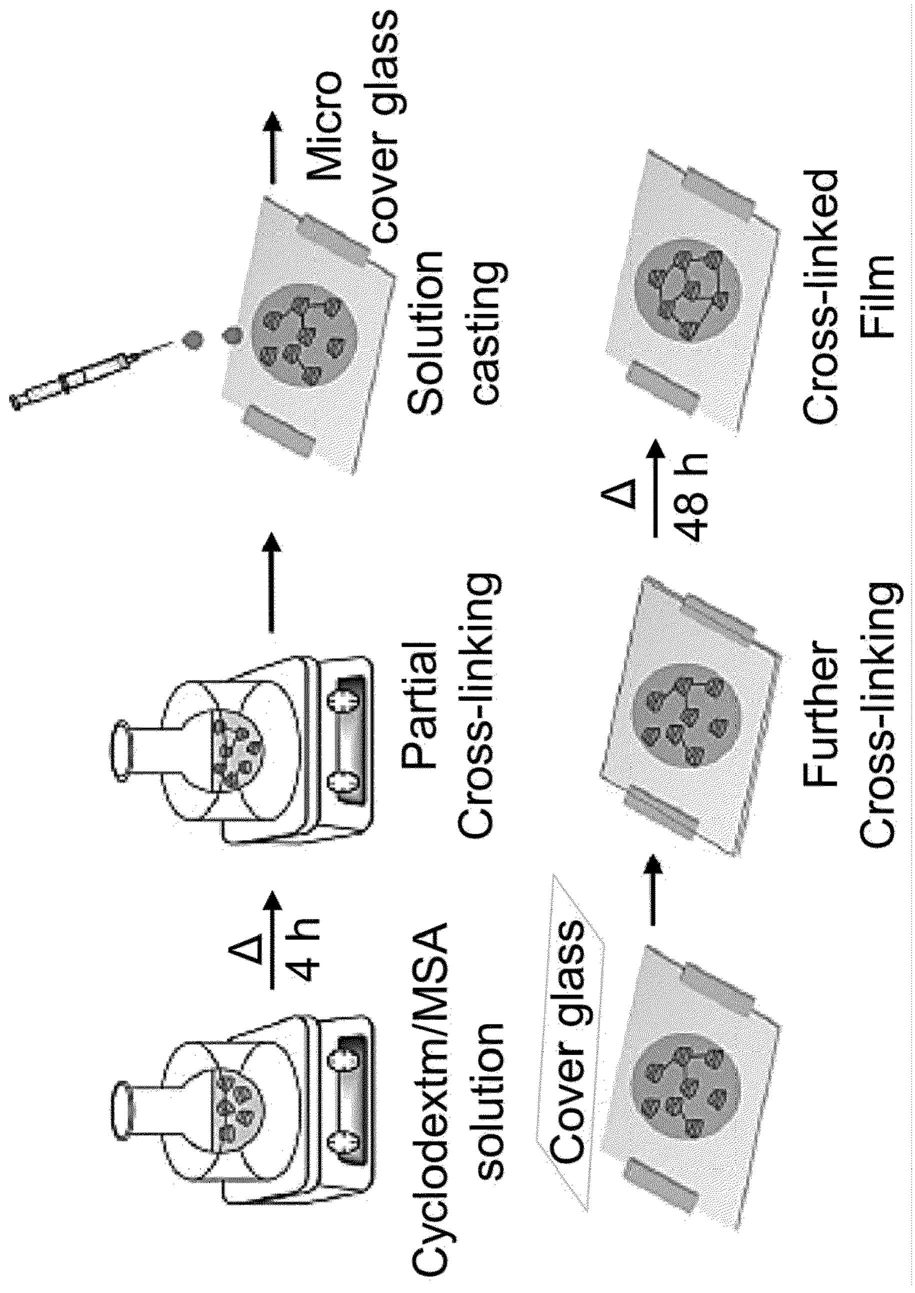
Cyclodextm/MSA solution
Δ
4 h
Partial Cross-linking
Solution casting
Micro cover glass
Cover glass
Further Cross-linking
Δ
48 h
Cross-linked Film

Figure 11

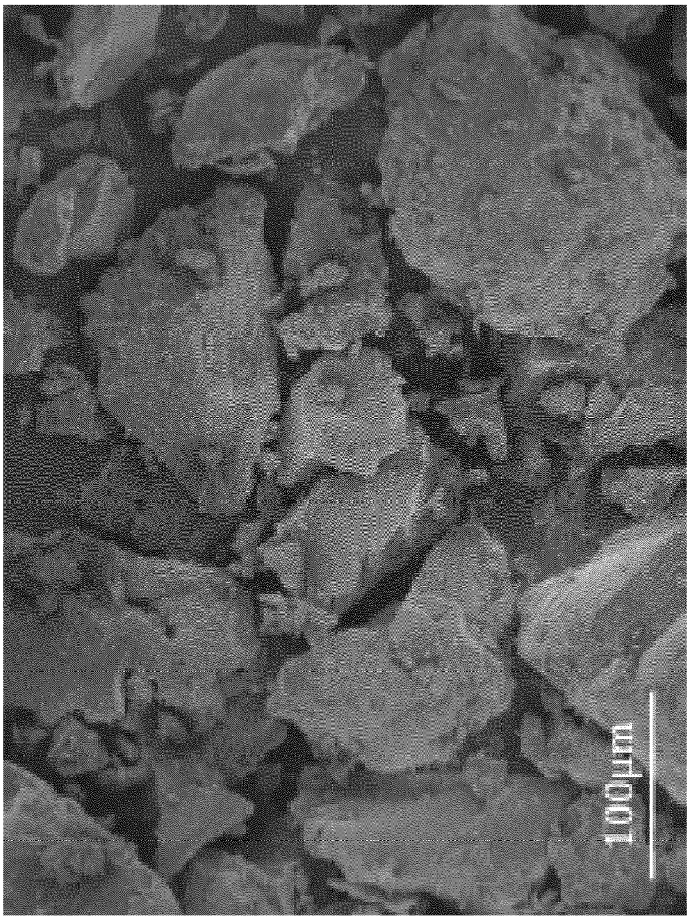


Figure 12

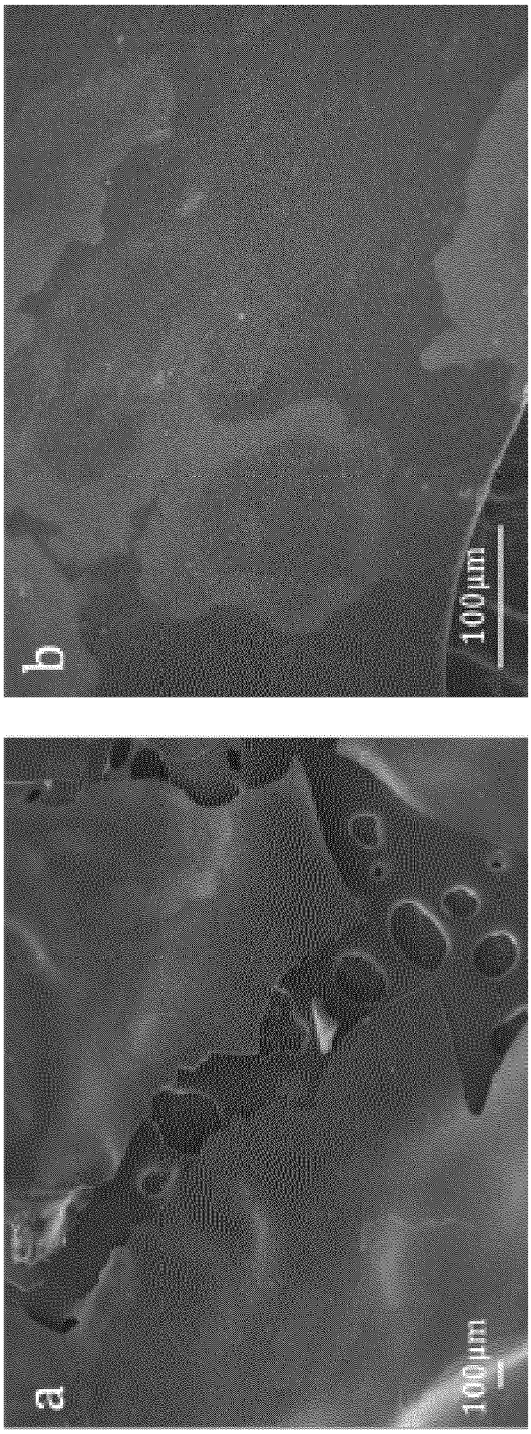


Figure 13

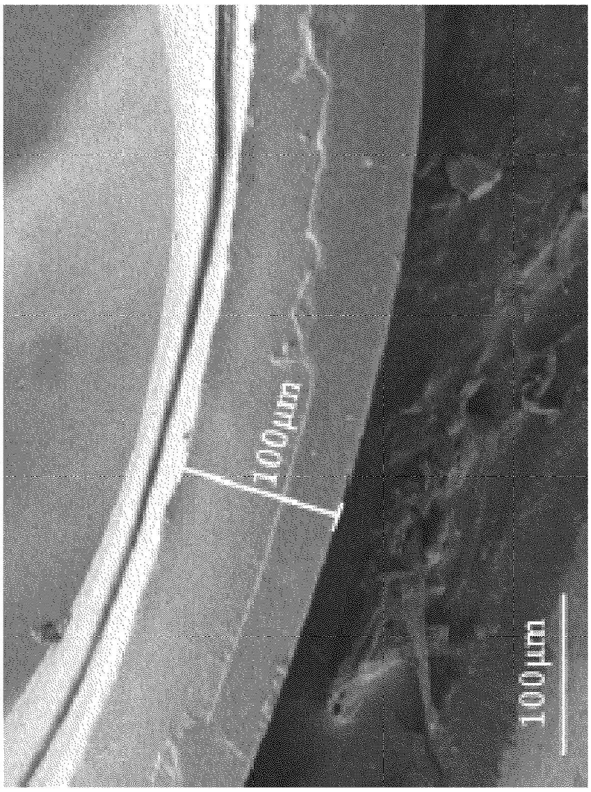


Figure 14

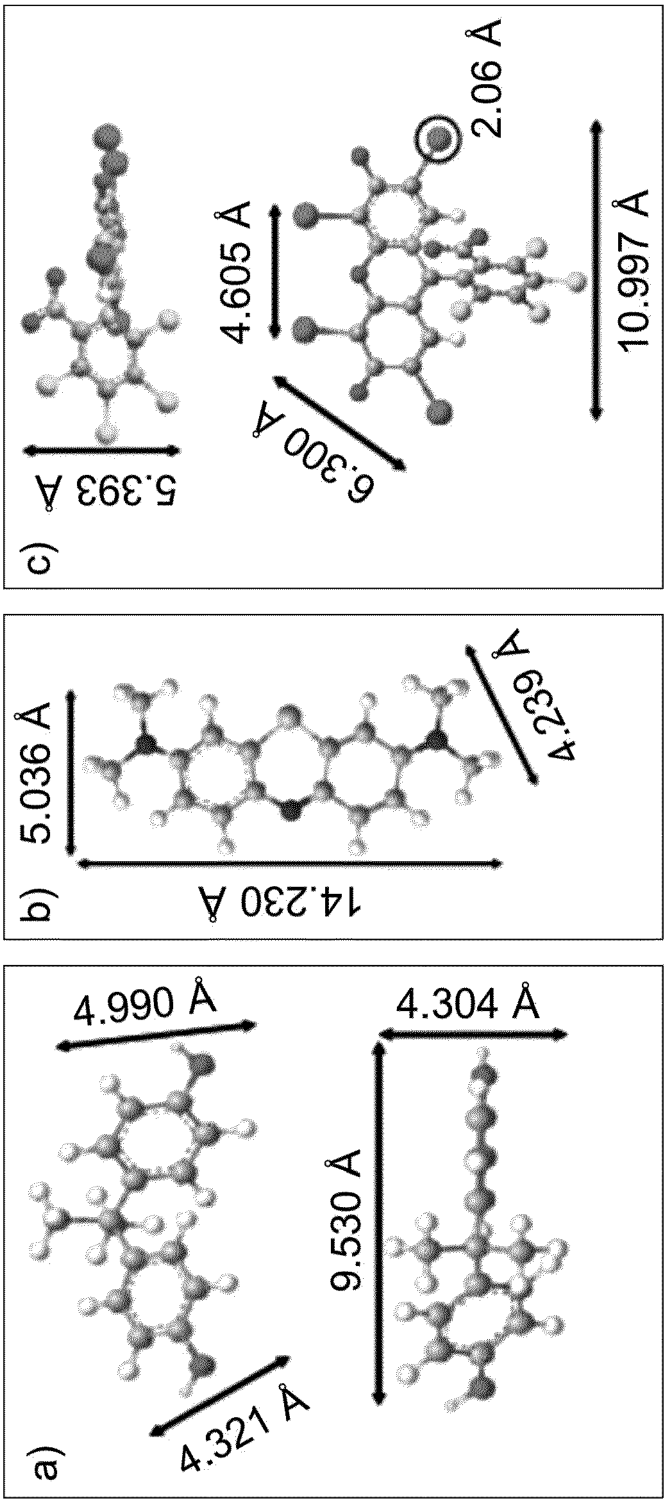
a)
4.990 Å
4.321 Å
4.304 Å
9.530 Å
b)
5.036 Å
14.230 Å
4.239 Å
c)
5.393 Å
6.300 Å
4.605 Å
10.997 Å
2.06 Å

Figure 15

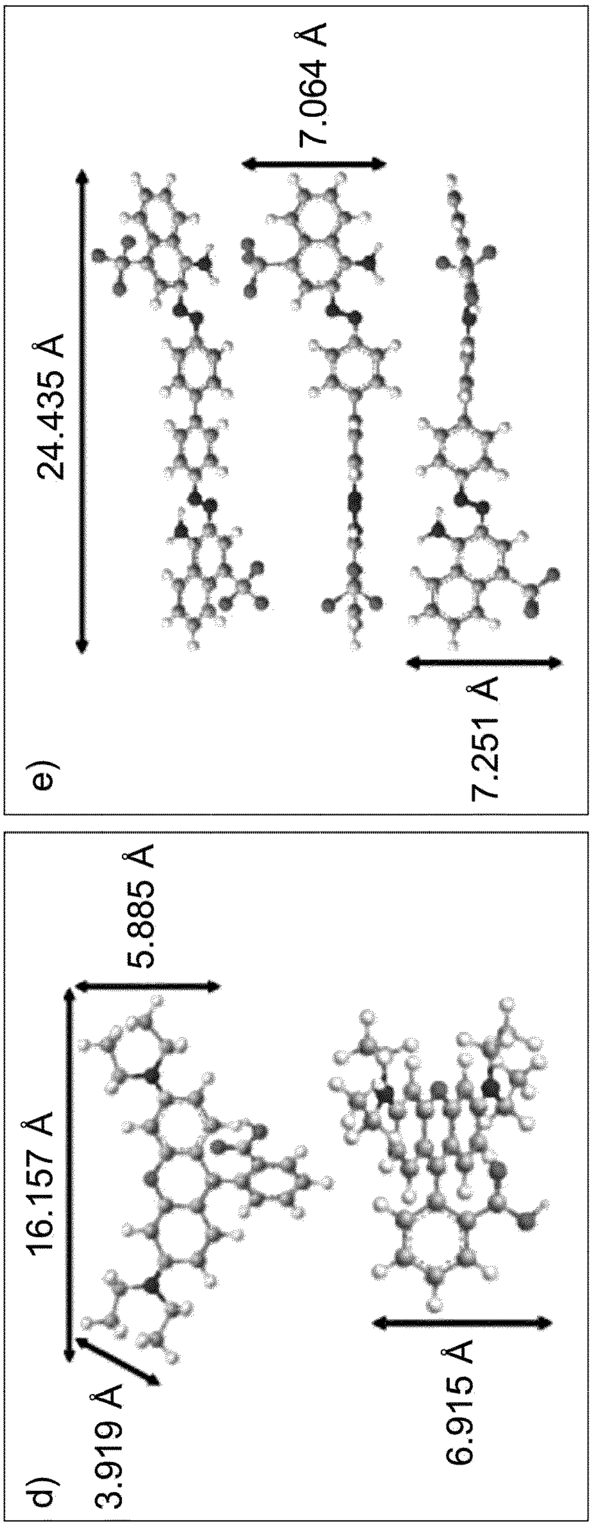


Figure 15
Continued

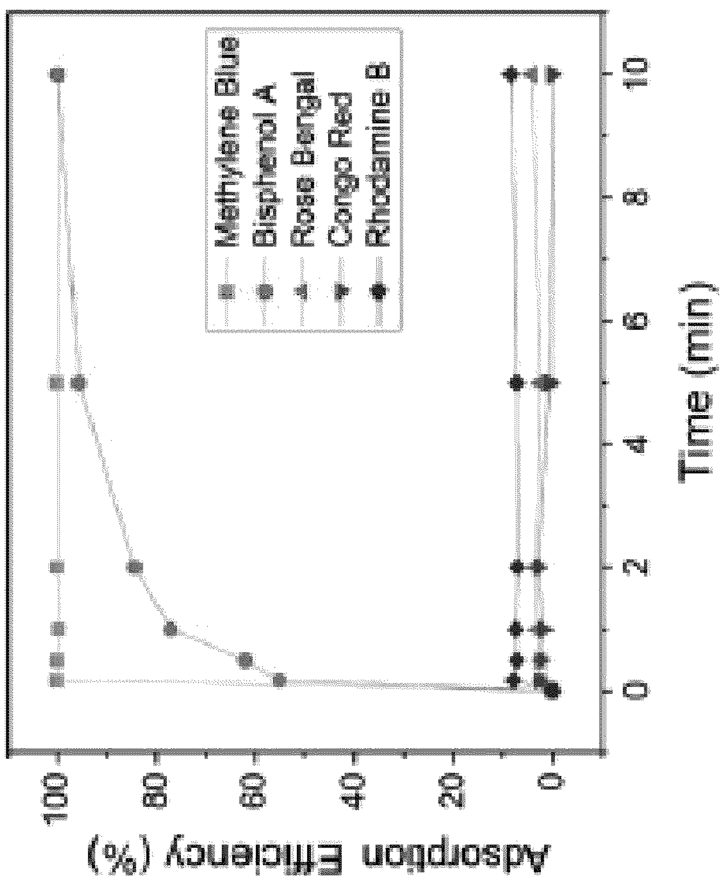
Methylene Blue
Bisphenol A
Rose Bengal
Congo Red
Rhodamine B
Adsorption Efficiency (%)
Time (min)
0
20
40
60
80
100
0
2
4
6
8
10

Figure 16

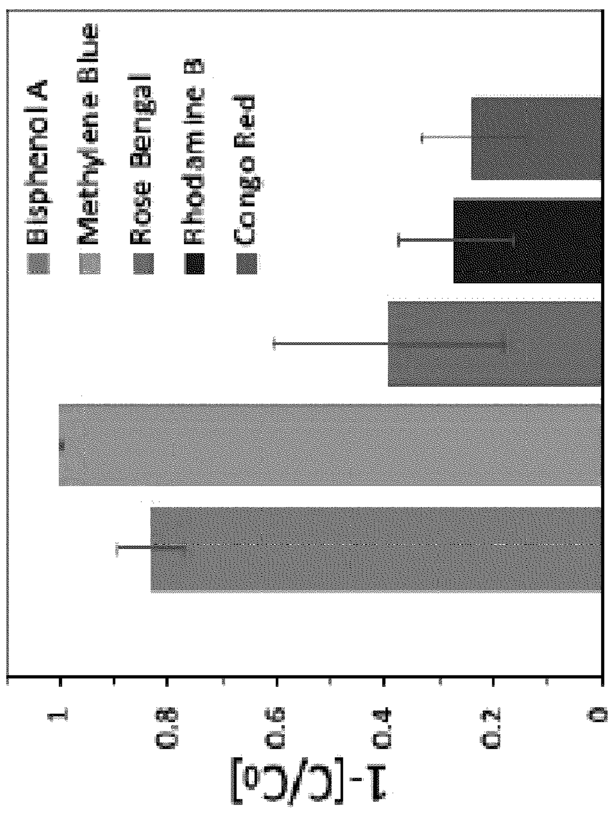
Bisphenol A
Methylene Blue
Rose Bengal
Rhodamine B
Congo Red
1
0.8
0.6
0.4
0.2
0
1-[C/C0]

Figure 17

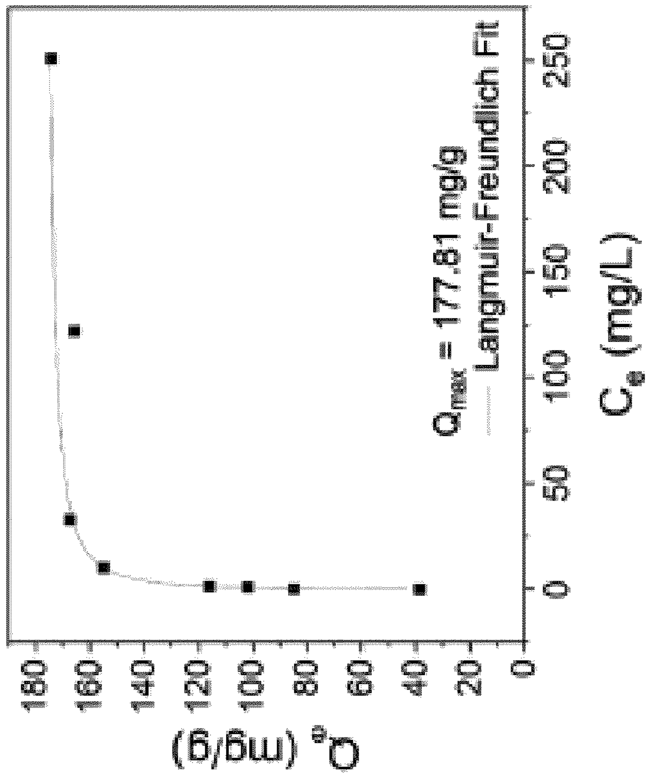
Q_max = 177.81 mg/g
Langmuir-Freundlich Fit
C_e (mg/L)
Q_e (mg/g)
0
50
100
150
200
250
0
20
40
60
80
100
120
140
160
180

Figure 18

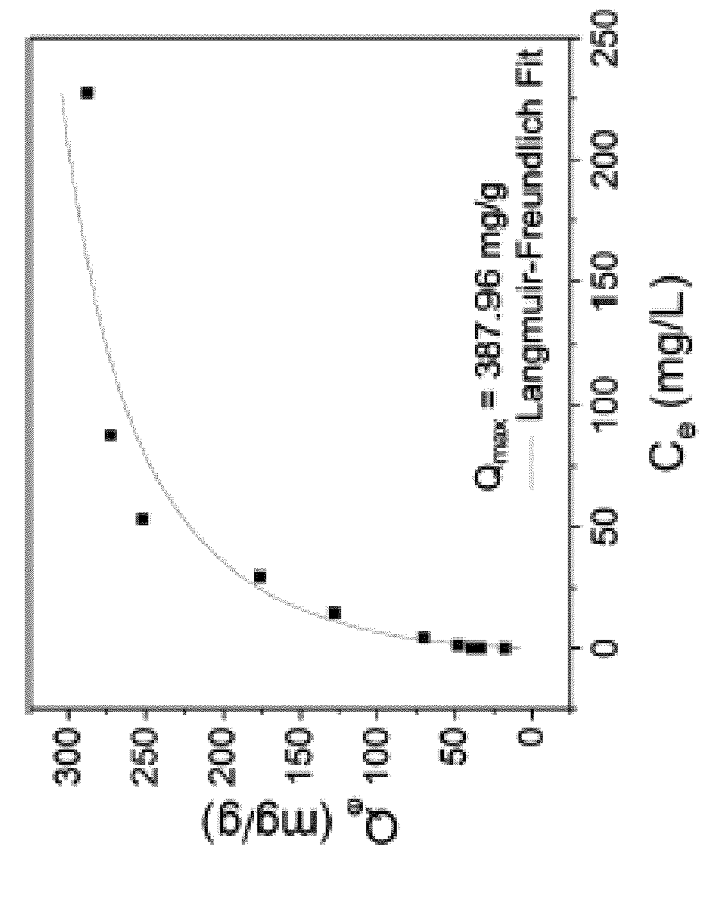
$Q_{max}$ = 387.96 mg/g
Langmuir-Freundlich Fit
$C_e$ (mg/L)
$Q_e$ (mg/g)
0
50
100
150
200
250
300

Figure 19

Europäisches Patentamt
European Patent Office
Office européen des brevets

# EUROPEAN SEARCH REPORT

Application Number
EP 22 16 7378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PHILLIPS BAILEY ET AL: "Cyclodextrin-derived polymer networks for selective molecular adsorption", CHEMICAL COMMUNICATIONS, vol. 56, no. 79, 6 October 2020 (2020-10-06), pages 11783-11786, XP055952646, UK ISSN: 1359-7345, DOI: 10.1039/D0CC04784H * paragraphs "2.Synthetic Procedures" and "8.Film Fabrication" * ----- | 1-15 | INV. C08B37/16 C07H1/00 C07H3/02 |
| X | CN 107 915 788 A (HU WEI) 17 April 2018 (2018-04-17) * example 3 * ----- | 1-6 | |
| | | | TECHNICAL FIELDS SEARCHED (IPC) |
| | | | C08B C07H |

The present search report has been drawn up for all claims

1

| Place of search | Date of completion of the search | Examiner |
|---|---|---|
| Munich | 18 August 2022 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 16 7378

This annex lists the patent family members relating to the patent documents cited in the above-mentioned European search report. The members are as contained in the European Patent Office EDP file on
The European Patent Office is in no way liable for these particulars which are merely given for the purpose of information.

18-08-2022

| Patent document cited in search report | Publication date | Patent family member(s) | Publication date |
|---|---|---|---|
| CN 107915788 A | 17-04-2018 | NONE | |

EPO FORM P0459

For more details about this annex : see Official Journal of the European Patent Office, No. 12/82